# EUROPEAN PATENT APPLICATION

(11) **EP 4 059 571 A1**
(43) Date of publication of application: **21.09.2022**
(21) Application number: 20875096.8
(22) Date of filing: 06.10.2020
(51) Int. Cl.: A61P 43/00, C12P 19/30, A23L 33/10, A61K 8/00, A61K 8/49, C12N 1/20, A61K 31/706

(54) **LACTIC ACID BACTERIA THAT PRODUCE NICOTINAMIDE RIBOSIDE, AND LACTIC ACID BACTERIA THAT PRODUCE NICOTINAMIDE MONONUCLEOTIDE AND NICOTINAMIDE RIBOSIDE**

(30) Priority: 11.10.2019 JP 2019187501
(71) Applicant: NATIONAL UNIVERSITY CORPORATION SHIZUOKA UNIVERSITY, Shizuoka-shi, Shizuoka 422-8529 (JP); Osaka Soda Co., Ltd., Osaka-shi Osaka 550-0011 (JP)
(72) Inventor: YOSHIDA, Nobuyuki, Hamamatsu-shi, Shizuoka 432-8561 (JP); NISHIKAWA, Kouji, Osaka-shi, Osaka 550-0011 (JP); IDOGAKI, Hideaki, Osaka-shi, Osaka 550-0011 (JP)
(74) Representative: Müller-Boré & Partner Patentanwälte PartG mbB
(86) International application number: PCT/JP2020/037902
(87) International publication number: WO 2021/070829

(57) **Abstract**

The purpose of the present invention is to provide microorganisms which efficiently produce nicotinamide riboside, and microorganisms which can efficiently produce both nicotinamide mononucleotide and nicotinamide riboside. Nicotinamide mononucleotide and nicotinamide riboside can be produced by culturing lactic acid bacteria belonging to the genus Fructobacillus.

## Description

### TECHNICAL FIELD

The present invention relates to a lactic acid bacteria that can produce nicotinamide riboside as well as a lactic acid bacteria that can produce nicotinamide mononucleotide and nicotinamide riboside.

### BACKGROUND ART

Aging and aging-related diseases have recently been shown to be closely involved with a decrease in the level of nicotinamide adenine dinucleotide (NAD⁺) and a decrease in the activity of an NAD⁺-dependent deacetylase sirtuin (Non-Patent Documents 1 and 2). In addition, it is considered that the sirtuin activation can explain the most of effects on the life extension or health promotion by caloric restriction (Non-Patent Document 2).

NAD⁺ has since long ago been known to be a coenzyme in a redox reaction. In recent years, NAD⁺ has also been known to serve as a substrate, etc., for poly-ADP ribose polymerase, CD38/CD157, or sirtuin (Non-Patent Document 1). A sirtuin-mediated NAD⁺ decomposition into nicotinamide can promote sirtuin-mediated deacetylation of a lysine residue. This participates in various life phenomena related to health and longevity (Non-Patent Documents 1 and 2). The level of NAD⁺ and the sirtuin activity are lowered as aging proceeds. Meanwhile, it has been known that when NAD⁺ metabolic intermediates such as enzyme reaction products of nicotinamide phosphoribosyltransferase (NAMPT), which is a rate-limiting enzyme for resynthesizing NAD⁺ from nicotinamide, specifically nicotinamide mononucleotide (NMN) and nicotinamide riboside (NR) are supplemented, sirtuin can be effectively reactivated (Non-Patent Document 1). It has further been known that the sirtuin activation can mediate expression of a wide range of antioxidant actions. In view of the above, nicotinamide mononucleotide and nicotinamide riboside have been known to contribute to many *in vivo* phenomena involving, for instance, the life extension and health promotion by caloric restriction.

Thus, a process for efficiently producing nicotinamide mononucleotide or nicotinamide riboside is desired. A previous report shows that nicotinamide mononucleotide can be obtained from edible yeast such as torula yeast (Patent Document 1). There is also a report showing that nicotinamide riboside can be obtained from genetically modified bacteria selected from the group consisting of *E*. *coli, B. subtilis, C. glutamicum, A. baylyi,* and *R. eutropha* (Patent Document 2).

### PRIOR ART DOCUMENTS

### PATENT DOCUMENTS

Patent Document 1: WO 2017/200050
Patent Document 2: WO 2017/083858

### NON-PATENT DOCUMENTS

Non-Patent Document 1: Imai, S. & Guarente, L. (2014) Trends Cell Biol., 24, 464-471.
Non-Patent Document 2: Guarente, L. (2013) Genes Dev., 27, 2072-2085.

### SUMMARY OF THE INVENTION

### PROBLEMS TO BE SOLVED BY THE INVENTION

There is still a room for improvement in production efficiency in a previous microbiological process for producing nicotinamide mononucleotide or nicotinamide riboside.

Accordingly, an object of the present invention is to provide a microorganism with increased nicotinamide riboside production efficiency and a microorganism that can produce both nicotinamide mononucleotide and nicotinamide riboside.

### MEANS FOR SOLVING THE PROBLEM

The present inventors have conducted intensive research and, as a result, have found that a lactic acid bacteria belonging to a specific genus can efficiently produce nicotinamide riboside and can further produce both nicotinamide mononucleotide and nicotinamide riboside. The present invention has been completed by conducting further studies based on the findings.

Specifically, the present invention provides the following aspects of the invention.

Item 1. A process for producing nicotinamide riboside, comprising the step of culturing a lactic acid bacteria belonging to the genus Fructobacillus to prepare a culture containing nicotinamide riboside.

Item 2. The production process according to item 1, wherein the culturing is performed in a fructose-free culture medium.

Item 3. The production process according to item 1 or 2, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 4. The production process according to item 1 or 2, wherein the lactic acid bacteria is *Fructobacillus tropaeoil* RD012353 strain (Deposit No: NITE P-02765) and/or *Fructobacillus fructosus* NBRC3516 strain.

Item 5. A nicotinamide riboside enhancer comprising a nicotinamide riboside-containing culture obtained from a lactic acid bacteria belonging to the genus Fructobacillus, a supernatant separated from the culture, and/or nicotinamide riboside isolated from the supernatant.

Item 6. The enhancer according to item 5, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 7. The enhancer according to item 5, wherein the lactic acid bacteria is *Fructobacillus tropaeoil* RD012353 strain (Deposit No: NITE P-02765) and/or *Fructobacillus fructosus* NBRC3516 strain.

Item 8. A food or drink, cosmetic, or pharmaceutical comprising the nicotinamide riboside enhancer according to any one of items 5 to 7.

Item 9. A nicotinamide riboside-producing lactic acid bacteria, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 10. A process for producing nicotinamide mononucleotide and nicotinamide riboside, comprising the step of culturing a lactic acid bacteria belonging to the genus Fructobacillus.

Item 11. The production process according to item 10, wherein the culturing is performed in a fructose-containing culture medium.

Item 12. The production process according to item 10 or 11, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 13. The production process according to item 10 or 11, wherein the lactic acid bacteria is *Fructobacillus tropaeoil* RD012353 strain (Deposit No: NITE P-02765) and/or *Fructobacillus fructosus* NBRC3516 strain.

Item 14. A nicotinamide mononucleotide and nicotinamide riboside enhancer comprising a nicotinamide mononucleotide- and nicotinamide riboside-containing culture obtained from a lactic acid bacteria belonging to the genus Fructobacillus, bacterial cell bodies separated from the culture, a homogenate of the bacterial cell bodies, and/or nicotinamide riboside and nicotinamide riboside isolated from the homogenate.

Item 15. The enhancer according to item 14, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 16. The enhancer according to item 14, wherein the lactic acid bacteria is *Fructobacillus durionis* RD011727 strain (Deposit No: NITE-P02764) and/or *Fructobacillus fructosus* NBRC3516 strain.

Item 17. A food or drink, cosmetic, or pharmaceutical comprising the nicotinamide mononucleotide and nicotinamide riboside enhancer according to any one of items 14 to 16.

Item 18. A nicotinamide mononucleotide- and nicotinamide riboside-producing lactic acid bacteria , wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

Item 19. Use of a nicotinamide riboside-containing culture obtained from a lactic acid bacteria belonging to the genus Fructobacillus, a supernatant separated from the culture, and/or nicotinamide riboside isolated from the supernatant in the manufacture of a nicotinamide riboside enhancer.

Item 20. Use of a nicotinamide mononucleotide- and nicotinamide riboside-containing culture obtained from a lactic acid bacteria belonging to the genus Fructobacillus, bacterial cell bodies separated from the culture, a homogenate of the bacterial cell bodies, and/or nicotinamide riboside and nicotinamide riboside isolated from the homogenate in the manufacture of a nicotinamide mononucleotide and nicotinamide riboside enhancer.

### ADVANTAGES OF THE INVENTION

The present invention can provide a lactic acid bacteria belonging to the genus Fructobacillus as a microorganism with increased nicotinamide riboside production efficiency, and as a microorganism that can produce both nicotinamide mononucleotide and nicotinamide riboside. Thus, a nicotinamide riboside-containing culture or a nicotinamide mononucleotide- and nicotinamide riboside-containing culture obtained from a lactic acid bacteria s belonging to the genus Fructobacillus can be used in a food supplement for reinforcing nicotinamide riboside or a food supplement for reinforcing nicotinamide mononucleotide and nicotinamide riboside, respectively.

### EMBODIMENTS OF THE INVENTION

### 1. Process for Producing Nicotinamide Riboside

A process for producing nicotinamide riboside according to the present invention comprises the step of culturing a lactic acid bacteria belonging to the genus Fructobacillus to prepare a culture containing nicotinamide riboside. Hereinafter, the nicotinamide riboside production process of the present invention will be described in detail.

Lactic acid bacteria A lactic acid bacteria used in the production process of the present invention is not particularly limited as long as the lactic acid bacteria belongs to the genus Fructobacillus. The lactic acid bacteria belonging to the genus Fructobacillus produces nicotinamide riboside.

Preferable examples of the lactic acid bacteria include Fructobacillus durionis, Fructobacillus tropaeoil, and Fructobacillus fructosus. More preferable examples include Fructobacillus tropaeoil and Fructobacillus fructosus.

More preferable examples of the lactic acid bacteria include *Fructobacillus durionis* RD011727 strain, *Fructobacillus tropaeoil* RD012353 strain, *Fructobacillus tropaeoil* RD012354 strain, *Fructobacillus fructosus* NBRC3516 strain, and *Fructobacillus durionis* NBRC113239 strain. Still more preferable examples of the lactic acid bacteria include *Fructobacillus durionis* RD011727 strain, *Fructobacillus tropaeoil* RD012353 strain, *Fructobacillus tropaeoil* RD012354 strain, and *Fructobacillus fructosus* NBRC3516 strain.

From the viewpoint of producing nicotinamide riboside in more quantities in the nicotinamide riboside production process of the present invention, the lactic acid bacteria is preferably *Fructobacillus fructosus* or *Fructobacillus tropaeoil* RD012353 strain, more preferably *Fructobacillus tropaeoil* RD012353 strain or *Fructobacillus fructosus* NBRC3516 strain, and particularly preferably *Fructobacillus tropaeoil* RD012353 strain.

*Fructobacillus durionis* RD011727 strain has been domestically deposited as Deposit No: NITE P-02764 on August 21, 2018 in the National Institute of Technology and Evaluation (NITE) Patent Microorganisms Depositary (NPMD) (Room 122, 2-5-8 Kazusakamatari, Kisarazu, Chiba, Japan).

*Fructobacillus tropaeoil* RD012353 strain has been domestically deposited as Deposit No: NITE P-02765 on August 21, 2018 in the NITE Patent Microorganisms Depositary (NPMD) (Room 122, 2-5-8 Kazusakamatari, Kisarazu, Chiba, Japan).

*Fructobacillus tropaeoil* RD012354 strain has been domestically deposited as Deposit No: NITE P-02766 on August 21, 2018 in the NITE Patent Microorganisms Depositary (NPMD) (Room 122, 2-5-8 Kazusakamatari, Kisarazu, Chiba, Japan).

One species of the Fructobacillus lactic acid bacteria s strains may be used singly, or multiple species may be used in combination.

### Culture medium

A culture medium used for culturing a Fructobacillus lactic acid bacteria can be selected, as appropriate. Examples of the culture medium include those (e.g., a preculture medium) used for expansion culture or those (e.g., a main culture medium) used for production culture. The main culture medium may be prepared using a basal medium used as a preculture medium by further including an additive(s). The form of the culture medium is preferably a liquid culture medium, but may be an agar broth. The culture medium typically contains, in addition to a carbon source, a nitrogen source and minerals.

Examples of the carbon source include a carbohydrate or sugar material. Examples of the carbohydrate include saccharides (e.g., a monosaccharide, disaccharide, oligosaccharide), polysaccharides, and sugar alcohols. Examples of the carbohydrate include lactose, sucrose, glucose, starch, xylitol, and dextrose. The sugar material may be an organic composition containing a carbohydrate. Examples thereof include a food such as a milk or processed milk product (e.g., nonfat dry milk, whey, milk powder, condensed milk), a soymilk or processed soymilk product (e.g., a soymilk hydrolysate), cereals, a fruit, or a vegetable. Examples of the milk include those derived from any mammal such as a cow, a goat, sheep, a buffalo, a camel, a llama, a donkey, a yak, a horse, or a reindeer. Note that the carbohydrate may be an isolated carbohydrate or may be included in a sugar material. The fructose (carbohydrate) used may be, for instance, included in the form of a fruit (sugar material). One kind of the carbon sources may be used singly, or multiple kinds thereof may be used in combination. Preferred is glucose among these carbon sources. In the nicotinamide riboside production process of the present invention, the culture medium is preferably free of fructose.

The concentration of carbon source in the culture medium is not particularly limited, and may be set, as appropriate, depending on, for instance, the type of culture medium and/or the culture protocol. The concentration is, for example, from 0.5 to 4 w/w%, preferably from 1 to 3 w/w%, and more preferably from 1.5 to 2.5 w/w%.

The nitrogen source used may be any of an inorganic or organic nitrogen source. Examples include: proteins such as yeast extract (e.g., brewer's yeast), meat extract, or casein; a protein hydrolysate such as peptone (e.g., protease peptone); a peptide compound such as peptides; or a nitrogen-containing salt such as an ammonium salt (e.g., ammonium citrate) or a nitrate. One kind of the nitrogen sources may be used singly, or multiple kinds thereof may be used in combination.

The concentration of nitrogen source in the culture medium is not particularly limited, and may be set, as appropriate, depending on, for instance, the type of culture medium and/or the culture protocol. In the case of proteins, the concentration is, for example, from 0.3 to 4 w/w%, preferably from 0.5 to 3 w/w%, and more preferably from 1 to 2 w/w%; in the case of a peptide compound, the concentration is, for example, from 0.1 to 2 w/w%, preferably from 0.3 to 1.8 w/w%, and more preferably from 0.5 to 1.5 w/w%; and in the case of a nitrogen-containing salt, the concentration is, for example, from 0.03 to 1.5 w/w%, preferably from 0.05 to 1 w/w%, and more preferably from 0.1 to 0.5 w/w%.

Examples of each mineral include manganese (e.g., manganese sulfate), zinc, iron, sodium (e.g., sodium acetate), potassium (e.g., dipotassium hydrogen sulfate, potassium phosphate), magnesium (e.g., magnesium sulfate), calcium, phosphorus (e.g., potassium phosphate), sulfur (e.g., manganese sulfate, potassium hydrogen sulfate, magnesium sulfate), or a trace element. One kind of the minerals may be used singly, or multiple kinds thereof may be used in combination. Among these minerals, manganese, sodium, magnesium, or potassium is preferable.

The concentration of mineral in the culture medium is not particularly limited, and may be set, as appropriate, depending on, for instance, the type of culture medium and/or the culture protocol. In the case of manganese, the concentration is, for example, from 0.001 to 0.01 w/w%, and preferably from 0.003 to 0.008 w/w%; in the case of sodium, the concentration is, for example, from 0.05 to 1.5 w/w%, and preferably from 0.1 to 1 w/w%; in the case of magnesium, the concentration is, for example, from 0.001 to 0.02 w/w%, and preferably from 0.005 to 0.015 w/w%; and in the case of potassium, the concentration is, for example, from 0.05 to 1 w/w%, and preferably from 0.1 to 0.5 w/w%.

The culture medium optionally contains, in addition to the above components, an additional component(s) such as a vitamin (e.g., a vitamin B group), a surfactant (e.g., a nonionic surfactant (e.g., Tween), an anionic surfactant (e.g., SDS)), an antibacterial (e.g., triclosan), and/or an antibiotic (e.g., monesin). One kind of the additional components may be used singly, or multiple kinds thereof may be used in combination. Among the additional components, preferred is a surfactant and more preferred is a nonionic surfactant.

The concentration of additional component(s) in the culture medium is not particularly limited, and may be set, as appropriate, depending on, for instance, the kind of the additional component(s), the type of culture medium and/or the culture protocol. In the case of including a surfactant, the concentration of surfactant is, for example, from 0.01 to 0.5 w/w%, and preferably from 0.05 to 0.3 w/w%.

In the case of using, as a food nicotinamide riboside enhancer, the culture obtained by the production process in the present invention, the culture medium component(s) selected may be a component(s) approved as a food additive(s) among the above components.

### Culturing Procedure

A procedure for culturing a lactic acid bacteria of the genus Fructobacillus in the production process of the present invention is not particularly limited as long as under conditions of growing a lactic acid bacteria of the genus Fructobacillus and under conditions in which a nicotinamide riboside-containing culture can be prepared.

The culturing temperature may be any temperature as long as the temperature is optimized for a lactic acid bacteria of the genus Fructobacillus to be cultured. The temperature is, for example, from 26 to 40°C, preferably from 27 to 38°C, more preferably from 28 to 36°C, and still more preferably from 29 to 34°C. The culturing period may be set, as appropriate, according to, for instance, the species of a lactic acid bacteria of the genus Fructobacillus to be actually cultured. The culturing period is, for instance, from 4 to 48 h, preferably from 8 to 36 h, and more preferably from 12 to 24 h.

In the production process of the present invention, the culture liquid is not necessarily stirred during culturing of a lactic acid bacteria of the genus Fructobacillus. Under such conditions, nicotinamide riboside can be produced abundantly. Note that in the production process of the present invention, the culture liquid may be stirred during culturing of a lactic acid bacteria in order to produce nicotinamide riboside more.

In the production process of the present invention, the above culturing is preferably performed as a production culture (main culture) for a certain period. Before that, an expansion culture (pre-culture) may be performed in a small volume of culture medium (e.g., a volume ratio of 1/6 to 1/4 of the culture medium for main culture). The culture conditions for the pre-culture may be set, as appropriate, according to, for instance, the species of a lactic acid bacteria of the genus Fructobacillus. Here, the above-described conditions may be adopted. In addition, in the main culture, the culture obtained through the pre-culture may be inoculated in the main culture medium so that OD660 is, for example, from 0.01 to 0.04 and preferably from 0.01 to 0.03.

The nicotinamide riboside-containing culture obtained as above can be prepared. This culture may contain other metabolites from a lactic acid bacteria of the genus Fructobacillus, which metabolites have been co-produced during culturing.

Examples of the use form of the nicotinamide riboside-containing culture prepared include a form in which the prepared culture is used as it is, a form in which a supernatant is separated from the culture and used, or a form in which nicotinamide riboside is isolated from the supernatant and used. Examples of the separation procedure include centrifugation or membrane filtration. Examples of the isolation procedure include each chromatography technique (e.g., ion exchange chromatography, gel filtration chromatography, reverse phase chromatography, high performance liquid chromatography), salting-out, or solvent precipitation.

### 2. Nicotinamide Riboside Enhancer; Food or Drink, Cosmetic, or Pharmaceutical

The nicotinamide riboside-containing culture, a supernatant separated from the culture, and/or nicotinamide riboside isolated from the supernatant, which are obtained as above, are preferably applicable as a nicotinamide riboside enhancer that can be blended as an active ingredient of a food supplement or a cosmetic or pharmaceutical for reinforcing nicotinamide riboside.

The nicotinamide riboside enhancer may be ingested, taken in, or applied while blended in a food or drink, a cosmetic, or a pharmaceutical. By doing so, the *in vivo* incorporated nicotinamide riboside can be converted to nicotinamide adenine dinucleotide, which is a substance that cells need for utilizing energy. This can elicit various useful effects.

The nicotinamide riboside enhancer may contain, in addition to nicotinamide riboside, other metabolites from a lactic acid bacteria of the genus Fructobacillus. In this case, the nicotinamide riboside enhancer may be used as an enhancer for nicotinamide riboside as well as at least any of the other metabolites from a lactic acid bacteria of the Fructobacillus.

The nicotinamide riboside enhancer may be blended in a food or drink, so that nicotinamide riboside is efficiently taken in by eating or drinking. As a result, the nicotinamide riboside intake will exert a health effect (e.g., caloric restriction, anti-aging, health promotion). Note that examples of the food or drink include not only a food or drink for humans, but also a diet for animals.

The food or drink is not particularly limited. Examples of the drink include, but are not particularly limited to, fermented milk (e.g., drink yogurt), a lactic acid bacteria beverage, a milk beverage (e.g., coffee milk, fruit milk), a tea beverage (e.g., green tea, black tea, oolong tea), a fruit/vegetable-based beverage (e.g., a beverage containing (e.g., orange, apple, or grape) fruit juice or (e.g., tomato and carrot) vegetable juice), an alcoholic beverage (e.g., beer, low-malt beer, wine), a carbonated beverage, a soft drink, or a water-based beverage; and examples of the food include a processed food such as fermented milk (e.g., set-type yogurt, soft yogurt), confectionery, an instant food, or seasoning.

In addition, examples of the food or drink include a functional food. The functional food means a food with a certain *in vivo* functionality. Examples thereof include a health functional food such as a food for specified health use (including a conditional "TOKUHO" food [a food for specified health use]) and a functional nutritional food, a function-indicating food, a food for special use, a dietary supplement, a health supplement, a supplement (e.g., in various dosage forms such as a tablet, a coated tablet, a dragee, a capsule, a liquid preparation), or a beauty food (e.g., a diet food). Furthermore, the functional food may be a special use food product such as a patient food, powdered milk for pregnant women and lactating women, powdered milk for infants, a food for the elderly, or a food for nursing care.

In addition, the nicotinamide riboside enhancer may be blended in a cosmetic. This application will exert a pharmacological effect (e.g., anti-aging) by nicotinamide riboside.

Examples of the cosmetic include a basic cosmetic (e.g., a lotion, an emulsion, a cream, an essence, a gel, a pack, a sheet mask, a lip cream); a skin cleanser (e.g., a facial cleanser, a makeup remover (including a cleansing agent), an exfoliating agent, a body shampoo); a body care cosmetic (e.g., a sunscreen agent, a body gel, a body massage agent, an antiperspirant, a deodorant, a hair remover, a bath additive); a makeup cosmetic (e.g., a foundation, powder, a lipstick, a cheek rouge, an eye shadow, an eyeliner, a mascara, an eyebrow ink); a nail cosmetic (e.g., a manicure, a nail remover); a hair cosmetic (e.g., a hair styling agent, a shampoo, a conditioner, a rinse, a hair tonic); or an oral cosmetic (e.g., a liquid dentifrice, a toothpaste, a mouth wash, a mouth spray).

Further, the nicotinamide riboside enhancer may be blended in a pharmaceutical. This intake or application may facilitate oral or transdermal intake of nicotinamide riboside as an active ingredient. By doing so, the nicotinamide riboside intake will exert a pharmacological effect (e.g., treatment or prophylaxis of, for instance, Cockayne's syndrome and aging-related diseases, treatment or prophylaxis of skin oxidative damage).

Examples of the pharmaceutical include an oral preparation or an external preparation (including a quasi-medicine). Examples of the dosage form of the oral preparation include a tablet, a coated tablet, a dragee, a capsule, or a liquid preparation. Examples of the dosage form of the external preparation include a liquid preparation (including, for instance, a lotion agent, a spray, an aerosol, or a milk solution), foams, an ointment, a cream, a gel, and a patch.

Examples of the subject that receives the food or drink, cosmetic, or pharmaceutical containing the nicotinamide riboside enhancer include a mammal. Examples include a primate (e.g., a human, a chimpanzee, a gorilla); a pet or livestock (e.g., a dog, a cat, a rabbit, a cow, a horse, a pig, a goat, sheep, a donkey); or an experimental animal (e.g., a mouse, a rat, a hamster, a monkey).

### 3. Process for Producing Nicotinamide Mononucleotide and Nicotinamide Riboside

A lactic acid bacteria belonging to the genus Fructobacillus also produces nicotinamide mononucleotide together with nicotinamide riboside. Thus, the present invention also provides a process for producing nicotinamide mononucleotide and nicotinamide riboside.

In the process for producing nicotinamide mononucleotide and nicotinamide riboside, culturing is performed under conditions of growing a lactic acid bacteria of the genus Fructobacillus and under conditions in which a culture containing nicotinamide mononucleotide and nicotinamide riboside can be prepared. In this way, nicotinamide mononucleotide and nicotinamide riboside can be co-produced. Thus, the culture containing nicotinamide mononucleotide and nicotinamide riboside can be prepared.

The lactic acid bacteria used in the process for producing nicotinamide mononucleotide and nicotinamide riboside is as described in the above "1. Process for Producing Nicotinamide Riboside". Specifically, preferable examples of the lactic acid bacteria include *Fructobacillus durionis, Fructobacillus tropaeoil,* or *Fructobacillus fructosus.*

More preferable examples of the lactic acid bacteria include *Fructobacillus durionis* RD011727 strain, *Fructobacillus tropaeoil* RD012353 strain, *Fructobacillus tropaeoil* RD012354 strain, *Fructobacillus fructosus* NBRC3516 strain, or *Fructobacillus durionis* NBRC113239 strain.

From the viewpoint of producing nicotinamide mononucleotide more abundantly (i.e., increasing the ratio of nicotinamide mononucleotide to nicotinamide riboside) in the process for producing nicotinamide mononucleotide and nicotinamide riboside according to the present invention, preferable examples of the lactic acid bacteria include *Fructobacillus durionis, Fructobacillus fructosus,* and *Fructobacillus tropaeoil* RD012353 strain. More preferable examples include *Fructobacillus durionis* and *Fructobacillus fructosus.* Still more preferable examples include *Fructobacillus durionis* RD011727 strain and *Fructobacillus fructosus* NBRC3516 strain.

From the viewpoint of producing both nicotinamide mononucleotide and nicotinamide riboside in a balanced manner in the process for producing nicotinamide mononucleotide and nicotinamide riboside according to the present invention, preferable examples of the lactic acid bacteria include *Fructobacillus tropaeoil.* Preferred is *Fructobacillus tropaeoil* RD012353 strain.

The culture medium used in the process for producing nicotinamide mononucleotide and nicotinamide riboside is as described in the above "1. Process for Producing Nicotinamide Riboside", except that fructose is further preferably included as a carbon source. The concentration of fructose in the culture medium is not particularly limited, and may be set, as appropriate, depending on, for instance, the type of culture medium and/or the culture protocol. The concentration is, for example, from 0.5 to 4 w/w%, preferably from 1 to 3.5 w/w%, and more preferably from 1.5 to 2.5 w/w%. The inclusion of fructose in the culture medium makes the lactic acid bacteria belonging to the genus Fructobacillus accumulate nicotinamide riboside and nicotinamide riboside in its bacterial cell body.

For instance, the culture protocol used in the process for producing nicotinamide mononucleotide and nicotinamide riboside is as described in the above "1. Process for Producing Nicotinamide Riboside". Accordingly, both nicotinamide mononucleotide and nicotinamide riboside can be produced. Examples of the use form of the prepared culture containing both nicotinamide mononucleotide and nicotinamide riboside include a form in which the prepared culture is used as it is, a form in which bacterial cell bodies are separated from the culture and used, a form in which the bacterial cell bodies are lysed and used, or a form in which nicotinamide riboside is isolated and used. Examples of the separation procedure include centrifugation and membrane filtration. Examples of the bacterial cell body lysis procedure include a bead-mediated lysis procedure. Examples of the isolation procedure include various chromatography techniques (e.g., ion exchange chromatography, gel filtration chromatography, reverse phase chromatography, high performance liquid chromatography), salting-out, and solvent precipitation.

### 4. Nicotinamide Mononucleotide and Nicotinamide Riboside Enhancer; Food or Drink, Cosmetic, or Pharmaceutical

The culture containing nicotinamide mononucleotide and nicotinamide riboside obtained in the process for producing nicotinamide mononucleotide and nicotinamide riboside, bacterial cell bodies separated from the culture, a homogenate of the bacterial cell bodies, and/or nicotinamide mononucleotide and nicotinamide riboside isolated from the homogenate are preferably applicable as a nicotinamide mononucleotide and nicotinamide riboside enhancer that can be blended as an active ingredient of a food supplement or a cosmetic or pharmaceutical for reinforcing nicotinamide mononucleotide and nicotinamide riboside.

The nicotinamide mononucleotide and nicotinamide riboside enhancer may be ingested, taken in, or applied while blended in a food or drink, a cosmetic, or a pharmaceutical. By doing so, the *in vivo* incorporated nicotinamide mononucleotide and nicotinamide riboside can be converted into nicotinamide adenine dinucleotide, which is a substance that cells need for utilizing energy. This can elicit various useful effects.

The nicotinamide mononucleotide and nicotinamide riboside enhancer may contain, in addition to nicotinamide mononucleotide and nicotinamide riboside, other metabolites from a lactic acid bacteria of the genus Fructobacillus. In this case, the nicotinamide mononucleotide and nicotinamide riboside enhancer can be used as an enhancer of, in addition to nicotinamide mononucleotide and nicotinamide riboside, the corresponding other metabolite(s) from a lactic acid bacteria of the genus Fructobacillus.

The nicotinamide mononucleotide and nicotinamide riboside enhancer may be blended in a food or drink, so that nicotinamide mononucleotide and nicotinamide riboside are efficiently taken in by eating or drinking.

The food or drink in which the nicotinamide mononucleotide and nicotinamide riboside enhancer is blended is as described in the above "2. Nicotinamide Riboside Enhancer; Food or Drink, Cosmetic, or Pharmaceutical."

In addition, the nicotinamide mononucleotide and nicotinamide riboside enhancer may be blended in a cosmetic. This application will exert a pharmacological effect by nicotinamide riboside and a pharmacological effect by nicotinamide mononucleotide (e.g., an increase in intracellular nicotinamide mononucleotide, anti-aging).

The cosmetic in which the nicotinamide mononucleotide and nicotinamide riboside enhancer is blended is as described in the above "2. Nicotinamide Riboside Enhancer; Food or Drink, Cosmetic, or Pharmaceutical."

Further, the nicotinamide mononucleotide and nicotinamide riboside enhancer for the food may be blended in a pharmaceutical. This intake or application may facilitate oral or transdermal intake of nicotinamide mononucleotide and nicotinamide riboside as an active ingredient. Accordingly, the nicotinamide mononucleotide intake will exert a pharmacological effect (e.g., treatment or prophylaxis of, for instance, aging-related diseases), and the nicotinamide riboside intake will exert a pharmacological effect (e.g., treatment or prophylaxis of, for instance, Cockayne's syndrome and aging-related diseases, treatment or prophylaxis of skin oxidative damage).

The dosage form and the application target of the pharmaceutical in which the nicotinamide mononucleotide and nicotinamide riboside enhancer is blended is as described in the above "2. Nicotinamide Riboside Enhancer; Food or Drink, Cosmetic, or Pharmaceutical".

### EXAMPLES

Hereinafter, the present invention will be described in more detail with reference to Examples. The present invention, however, is not limited to them.

### Example 1 To Produce Nicotinamide Riboside by Using lactic acid bacteria s of the Genus Fructobacillus.

*Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain were each independently inoculated in 3 ml of MRS broth (preculture medium), manufactured by Difco, Inc., and were each subjected to static expansion culture at 30°C for 24 hours. The resulting culture liquid was inoculated in 15 ml of MRS broth (main culture medium), so that OD660 was 0.02. The culture was then subjected to static production culture at 30°C for 12 hours. The resulting culture liquid was centrifuged to separately collect a supernatant and the bacterial cell bodies. Nicotinamide mononucleotide (NMN) and nicotinamide riboside (NR) of the culture supernatant collected or the bacterial cell bodies collected were quantified.

### (MRS Broth Composition)

2 w/w% Glucose
1 w/w% Protease peptone
1 w/w% Beef extract
0.5 w/w% Yeast extract
0.2 w/w% Ammonium citrate
0.1 w/w% Tween 80
0.5 w/w% Sodium acetate
0.01 w/w% Magnesium sulfate
0.005 w/w% Manganese sulfate
0.2 w/w% Dipotassium hydrogen phosphate

### (To Quantify Nicotinamide Mononucleotide and Nicotinamide Riboside in Supernatant

Here, 15 ml of the supernatant collected and the same volume of 4 w/w% sodium bicarbonate solution were mixed. The resulting mixture was made to pass through a BondElut PBA column (1ml; manufactured by Agilent). Then, 3 ml of 2 w/w% sodium bicarbonate solution was further passed through the column for washing. Subsequently, 3 ml of 2 v/v% formic acid solution was passed through the column to recover an eluate. After 150 µl of 1.3 M aqueous potassium hydroxide solution and 100 µl of 20 w/w% acetophenone were added to 250 µl of the eluate, the mixture was reacted at 4°C for 30 min. Thereafter, 400 µl of 98 v/v% aqueous formic acid solution was added, and the mixture was then reacted at 110°C for 7 min. In this way, nicotinamide mononucleotide and nicotinamide riboside were subjected to fluorescence derivatization, and analyzed under HPLC conditions to be described later. Finally, the amount (the production amount per culture liquid; mg/L) of nicotinamide mononucleotide and nicotinamide riboside produced in the supernatant was measured. Table 1 shows the results. Note that the "n.d." in Table 1 represents "not detected".

### (To Quantify Nicotinamide Mononucleotide and Nicotinamide Riboside in Bacterial Cell Bodies)

The bacterial cell bodies collected were washed with 10 ml of 0.85 w/w% KCI solution. The washed bacterial cell bodies were re-centrifuged to collect the bacterial cell bodies. The bacterial cell bodies collected were suspended in 0.5 ml of 0.1 M potassium phosphate buffer (pH 7.0). The equal volume of 0.1-mm zirconia beads were added, and the bacterial cell bodies were then homogenized with a bead crusher. The homogenized bacterial cell bodies were centrifuged to collect a supernatant (bacterial cell body homogenate extract). Here, 150 µl of 1.3 M aqueous potassium hydroxide solution and 100 µl of 20 w/w% acetophenone were added to 250 µl of the bacterial cell body homogenate extract, and the mixture was reacted at 4°C for 30 min. Thereafter, 400 µl of 98 v/v% aqueous formic acid solution was added, and the mixture was then reacted at 110°C for 7 min. In this way, nicotinamide mononucleotide and nicotinamide riboside were subjected to fluorescence derivatization, and analyzed under HPLC conditions to be described later. Finally, the amount (the production amount per bacterial cell body; mg/1 g dry bacterial cell body) of nicotinamide mononucleotide and nicotinamide riboside produced in the collected bacterial cell bodies was measured. Table 1 shows the results.

### (HPLC analysis conditions)

Column: YMC Triart C 18 (4.6 x 150 mm)
Column temperature: 30°C
Eluent A: 0.1 v/v% aqueous formic acid solution
Eluent B: 0.1 v/v% formic acid-containing acetonitrile solution
Gradient condition: gradient from 0 min (eluent A:eluent B = 9:1 (volume ratio)) to 15 min (eluent A:eluent B = 3:7 (volume ratio))
Flow rate: 1 ml/min
Detector: fluorescence detector (Ex: 320 nm, Em: 458 nm)
Detection time: 4.3 min (for nicotinamide mononucleotide) and 5.1 min (for nicotinamide riboside)

**[Table 1]**

| | In supernatant (mg/L) | | In bacterial cell bodies (mg/1 g dry bacterial cell body) | |
|---|---|---|---|---|
| | NMN | NR | NMN | NR |
| Fructobacillus durionis RD0 11 72 7 strain | n.d. | 0.8 | n.d. | n.d. |
| Fructobacillus tropaeoil RD012353 strain | n.d. | 1.5 | n.d. | n.d. |
| Fructobacillus tropaeoil RD012354 strain | n.d. | 0.8 | n.d. | n.d. |
| Fructobacillus fructosus NBRC3516 strain | n.d. | 1.1 | n.d. | n.d. |

As clearly shown in Table 1, in a culture obtained using a fructose-free culture medium, neither nicotinamide mononucleotide (NMN) nor nicotinamide riboside (NR) was detected; and in the supernatant, although no nicotinamide mononucleotide (NMN) was detected, nicotinamide riboside (NR) was found to be produced specifically in the supernatant. Here, among them, *Fructobacillus tropaeoil* RD012353 strain and *Fructobacillus fructosus* NBRC3516 strain, in particular, *Fructobacillus tropaeoil* RD012353 strain was found to be able to produce nicotinamide riboside (NR) abundantly.

### Example 2 To Produce Nicotinamide Mononucleotide and Nicotinamide Riboside by Using Lactic acid bacteria of the Genus Fructobacillus

The same procedure as in Example 1 was repeated, except that 2 w/w% fructose was added to the main culture medium, to culture and collect the bacterial cell bodies and the culture supernatant and then quantify nicotinamide mononucleotide and nicotinamide riboside. Table 2 shows the results.

**[Table 2]**

| | In supernatant (mg/L) | | In bacterial cell bodies (mg/1 g dry bacterial cell body) | |
|---|---|---|---|---|
| | NMN | NR | NMN | NR |
| Fructobacillus durionis RD011727 strain | n.d. | n.d. | 1.8 | 0.07 |
| Fructobacillus tropaeoil RD012353 strain | n.d. | n.d. | 1.4 | 0.19 |
| Fructobacillus tropaeoil RD012354 strain | n.d. | n.d. | 0.76 | 0.06 |
| Fructobacillus fructosus NBRC3516 strain | n.d. | n.d. | 1.4 | 0.09 |

As clearly shown in Table 2, in a culture obtained using a fructose-containing culture medium, neither nicotinamide mononucleotide (NMN) nor nicotinamide riboside (NR) was detected in the supernatant; but nicotinamide mononucleotide (NMN) and nicotinamide riboside (NR) were found to be produced specifically in the bacterial cell bodies. In addition, *Fructobacillus tropaeoil* RD012353 strain and *Fructobacillus tropaeoil* RD012354 strain, in particular, *Fructobacillus tropaeoil* RD012353 strain produced both nicotinamide mononucleotide (NMN) and nicotinamide riboside (NR) in a balanced manner. Meanwhile, *Fructobacillus durionis* RD011727 strain and *Fructobacillus fructosus* NBRC3516 strain, in particular, *Fructobacillus durionis* RD011727 strain produced both nicotinamide mononucleotide (NMN) and nicotinamide riboside (NR). Of the two, nicotinamide mononucleotide (NMN) was found to be produced at a particularly high ratio.

## Claims

1. A process for producing nicotinamide riboside, comprising the step of culturing a lactic acid bacteria belonging to the genus Fructobacillus to prepare a culture containing nicotinamide riboside.

2. The production process according to claim 1, wherein the culturing is performed in a fructose-free culture medium.

3. The production process according to claim 1 or 2, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

4. The production process according to claim 1 or 2, wherein the lactic acid bacteria is *Fructobacillus tropaeoil* RD012353 strain (Deposit No: NITE P-02765) and/or *Fructobacillus fructosus* NBRC3516 strain.

5. A nicotinamide riboside enhancer comprising a nicotinamide riboside-containing culture obtained from a lactic acid bacteria belonging to the genus Fructobacillus, a supernatant separated from the culture, and/or nicotinamide riboside isolated from the supernatant.

6. The enhancer according to claim 5, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

7. The enhancer according to claim 5, wherein the lactic acid bacteria is *Fructobacillus tropaeoil* RD012353 strain (Deposit No: NITE P-02765) and/or *Fructobacillus fructosus* NBRC3516 strain.

8. A food or drink, cosmetic, or pharmaceutical comprising the nicotinamide riboside enhancer according to any one of claims 5 to 7.

9. A nicotinamide riboside-producing lactic acid bacteria, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

10. A process for producing nicotinamide mononucleotide and nicotinamide riboside, comprising the step of culturing a lactic acid bacteria belonging to the genus Fructobacillus.

11. The production process according to claim 10, wherein the culturing is performed in a fructose-containing culture medium.

12. The production process according to claim 10 or 11, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

13. The production process according to claim 10 or 11, wherein the lactic acid bacteria is *Fructobacillus tropaeoil* RD012353 strain (Deposit No: NITE P-02765) and/or *Fructobacillus fructosus* NBRC3516 strain.

14. A nicotinamide mononucleotide and nicotinamide riboside enhancer comprising a nicotinamide mononucleotide- and nicotinamide riboside-containing culture obtained from a lactic acid bacteria belonging to the genus Fructobacillus, bacterial cell bodies separated from the culture, a homogenate of the bacterial cell bodies, and/or nicotinamide riboside and nicotinamide riboside isolated from the homogenate.

15. The enhancer according to claim 14, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.

16. The enhancer according to claim 14, wherein the lactic acid bacteria is *Fructobacillus durionis* RD011727 strain (Deposit No: NITE-P02764) and/or *Fructobacillus fructosus* NBRC3516 strain.

17. A food or drink, cosmetic, or pharmaceutical comprising the nicotinamide mononucleotide and nicotinamide riboside enhancer according to any one of claims 14 to 16.

18. A nicotinamide mononucleotide- and nicotinamide riboside-producing lactobacillus, wherein the lactic acid bacteria is selected from the group consisting of *Fructobacillus durionis* RD011727 strain (Deposit No: NITE-P02764), *Fructobacillus tropaeoil* RD012353 strain (Deposit No: NITE P-02765), *Fructobacillus tropaeoil* RD012354 strain (Deposit No: NITE P-02766), and *Fructobacillus fructosus* NBRC3516 strain.
